# EUROPEAN PATENT APPLICATION

(11) **EP 1 944 022 A1**
(43) Date of publication of application: **16.07.2008**
(21) Application number: 06783793.0
(22) Date of filing: 23.06.2006
(51) Int. Cl.: A61K 31/137, A61K 31/195, A61P 25/04, A61P 25/08, A61P 29/00

(54) **PHARMACEUTICAL COMPOSITIONS COMBINING ANALGESICS AND ANTICONVULSANT AGENTS FOR THE TREATMENT OF CHRONIC AND ACUTE PAIN**

(30) Priority: 01.11.2005 MX PA05/01173
(71) Applicant: ESPINOZA ABDALA, Leopoldo de Jesús, Jalisco Mexico (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco (MX); ÁLVAREZ OCHOA, Víctor Guillermo, C.P.45222 Zapopan, Jalisco (MX); SANTOS MURILLO, Josefina, C.P. 44600 Zapopan, Jalisco (MX)
(74) Representative: Carvajal y Urquijo, Isabel
(86) International application number: PCT/MX2006/000059
(87) International publication number: WO 2007/052999

(57) **Abstract**

The present invention relates to pharmaceutical industry in general, and to pharmaceutical industry making medicaments for treatment of conditions of sharp and chronic pain. The composition is **characterized in that** it comprises a combination of an analgesic and an anticonvulsant, wherein the analgesic is known as Tramadol and the anticonvu.lsant drug is known as Gabapentin, wherein Tramadol is present in a rate with respect to the total weight of the formulation of from 3 to 8.3% and Gabapentin is present in a rate with respect to the total weight of the formulation of from 16 to 83%.

## Description

### FIELD OF THE INVENTION

The present invention relates to a pharmaceutical composition comprising a combination of an analgesic known as Tramadol and an anticonvulsant drug known as Gabapentin and to pharmacological use of the composition in treatment of conditions of sharp and chronic pain; the composition produces a combination product with improved properties, which requires a fewer amount of each ingredient and causes a synergic effect.

### BACKGROUND OF THE INVENTION

Pain is the most common cause among patients for medical treatment. Sharp and chronic pain is weakening, its recovery is slow, interferes with daily activities, and manifested as a decline in the life quality of patients.

Sharp pain is often associated with antecedent of an identifiable lesion or trauma, responds to therapeutic options.and solved in less than 1 to 3 months. Chronic pain is a challenge for management because the pathogenesis is not often clear, with less opportunity to predict the course of recovery. For patients who are already in a financial and psychological stress, as well as in an uncertain prognosis this is desolating.

Chronic pain is defined as that exceeding 3 months duration, it can be continuous or episodic, or both.

Chronic pain is accompanied by emotional stress, increasing irritability, depression, social restraint, financial distress, loss of libido, sleep disturbances, decrease of appetite and/or weight loss.

Examples of these pains are neuropathic, post-herpetic pain, diabetic neuropathy, alcoholic neuropathy, cancer pain, trigeminal neuralgia, fibromyalgia.

The pharmacological plan begins when the type of pain and its origin (sharp or chronic, nociceptive or neuropathic) has been identified.

There, is a wide amount of pharmacological agents for pain management; however, it should be considered the concomitant administration of different medicaments used for pain, to achieve an analgesic efficacy through different mechanisms of action, achieve a reduction in concentrations of active ingredients, increase the analgesic synergy and reduce the adverse events.

Tramadol is a phenyl-substituted aminomethyl-cyclohexane derivative, and is chemically (±) *cis*-2-[(dimethylamino)methyl]-1-(3-metoxyphenyl) cyclohexanol and corresponds to formula:

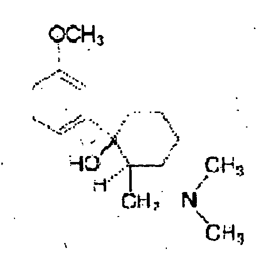

Tramadol is an agent that acts centrally and is generally classified as an opioid analgesic, but its mechanism of action is not fully understood. It is an efficacious and potent analgesic, comparable to codeine, pentazocine or dextropropoxifen. Of particular importance are the findings regarding the fact that tramadol does not accelerates the abstinence syndrome (Friederich et al., 1978), involving it as a pure opioid agonist and without antagonist activity. Clinical practice has shown that tramadol is unique among the potent analgesics that act centrally. Therapeutic use of tramadol has not been associated with significant side effects such as respiratory suppression, constipation (even with long-term administration) or sedation (Lehmann et al., 1990). There is evidence that tramadol exhibits low affinity to opioid receptors and little selectivity to sites linking to µ - κ -, or δ [Hennies et al., 1985, 1988] or shows a moderate selectivity µ when the most specific ligands are used Raffa et al., 1992].

Spinally and supraspinally, tramadol is associated with a low extent of respiratory suppression, as compared to the other opioids. It has a low potential for tachyphylaxis and misuse, and is used in long-term treatment of moderate to severe pain. The opioids have been used for many years as analgesics for treating severe pain; however, they produce undesirable adverse effects and as a result cannot always be administered iteratively or at high doses. Within the adverse effects that have been reported, and especially with the tramadol administration in high doses (100 mg) to Mexican patients, are nausea, vomit and sedation. To reduce the side effects of opioids, these have been combined with other drugs, including non-opioid analgesic agents, which reduce the opioid amount needed to produce an equivalent extent of analgesia, and it has been reported that some of those combined products have the advantage of require a fewer amount of each ingredient while produce an synergic analgesic effect.

Compositions including combinations of analgesic opioids with other drugs, such as analgesics, exhibit a variety of sub-additive (inhibitory), additive or superadditive effects (A. Takemori, Annals New York. Acad. Sci., 1976, 281, 262).

As an analgesic, tramadol has been combined with other opioid and non-opioid analgesics. These combinations have shown synergic effects in pain treatment while fewer amounts of ingredients, which produce an equivalent extent of analgesia, are used. Specifically, U.S. Patent No. 5,516,803 discloses the composition of tramadol and an AINE, particularly ibuprofen. The U.S. Patent No. 5,468,744 discloses a tramadol plus any of the following, oxycodone, codepine or hydrocodone, and the U.S. Patent No. 5, 336, 691 discloses tramadol in combination with acetaminophen.

As a class, the anticonvulsants are medicaments unknown by its usefulness in treatment of neuropathic pain. However, certain anticonvulsants have been found to be useful in treatment of neuropathic pain, as in the U.S.. Patent No. 4,513,006, which discloses a class of anticonvulsants including 2,3,4,5-bis-O-(1-methylethylidene)-beta-D-Fructopyranose sulfamates also known as topiramate. U.S. Patent No. 5,760,007, incorporated herein by reference, discloses topiramate for treatment of neuropathic pain.

In addition, anticonvulsants have been combined with non-toxic blockers for the N-methyl-d-aspartate (NMDA) receptor. These compositions have been described as useful in treatment of neuropathic pain. For example, WO 98/07447 widely discloses the combination of an amount of anticonvulsant to relieve neuropathic pain, including gabapentin, lamotrigine, valproic acid, topiramate, famotidine, phenobarbital, diphenylhydantoin, phenytoin, mephenytoin, ethotoin, mephobarbital, primidone, carbamazepine, ethosuximide, metsuximide, phensuximide, trimetadione, benzodiazepine, phenacemide, acetazolamide, progabide, clonazepam, divalproex sodium, sodium valproate, clobazam, sultiame, dilantin, difenilan or L-5-hydroxytryptophan and an anticonvulsant, which enhance the amount of a receptor blocker, non-toxic to the NMDA. This reference, however, does not show the synergic composition of the present invention.

Anticonvulsants have also been combined with AINE's or narcotic analgesics described as useful in treatment of pain. WO 99/12537 describes a composition of the anticonvulsant gabapentin or pregabalin in combination with the AINE-naproxen or narcotic analgesics. Combinations of anticonvulsants and other medicaments, such as opioid analgesics, have been suggested (Donnadieu, S., et al., Pain Relief, Presse Medicale, 1998, 27/39, 2062-2069). This reference, however, does not show the synergic composition of the present invention.

None of the references has described a pharmaceutical combination comprising a combination of an analgesic that acts centrally, such as tramadol, and an anticonvulsant such as gabapentin, which shows that the composition has a synergic effect with a fewer amount of ingredients for treating conditions of sharp and chronic pain in mammals and humans.

Gabapentin is indicated for treatment of partial seizure in adults and children older than 12 years old; however, it has been used for management of chronic pain especially in patients with neuropathic pain, and has been considered as the first-line treatment. It is structurally related to neurotransmitter (gamma-aminobutyric), but its mechanism of action is different from that of many drugs that have GABA synapsis. Recent studies have suggested that gabapentin may have a role in management of severe forms of chronic pain. The modulation of the GABA-ergic system would be a promissory treatment for these neuropathic disorders.

The object of the present invention is to produce a combination product with tramadol and gabapentin, which improves its analgesic properties. It is also an object of this invention to produce a combination product which has a synergic effect while a fewer amount of each ingredient is used, which provides a method for treating conditions of sharp and chronic pain in mammals and humans with fewer side effects.

### DETAILED DESCRIPTION OF THE INVENTION

The associations of analgesic agents containing two or more compounds are widely used in therapeutics; many of these associations are formulated in an attempt to produce greater analgesic effects by a synergic activity, con fewer amount of each ingredient and fewer side effects. Example 1 shows the synergic activity of the combination in nociceptive pain.

### Example 1:

An experimental preclinical study was performed that compares the analgesic effect of tramadol and gabapentin administered individually and in combination, using female Wistar rats weighting from 160 to 180 g, which were intraarticularly injected 0.05 ml uric acid suspended in mineral oil into knee joint of left hindleg under anesthesia in order to cause inflammation and pain. An electrode was connected at the plantar region of hindlegs. The rats were placed in a roller of 30 cm of diameter rotating at 4 rpm during two-minute periods. The time of contact between each electrode of the rats' hindlegs and the roller was registered. The data are expressed as Functionality Ratio (FR). This is equivalent to the time of contact of the swollen and painful leg and the control of right leg without pain multiplied by 100. The functionality ratio reached zero after two hours of intraarticular administration of uric acid. At the same time the medicaments, alone or in combination, were administered, and the Functionality Ratio every 30 minutes for 4 hours was determined. The analgesia was estimated as the recovery of the Functionality Ratio (FR). The accumulated analgesic effect during the observation period (4 hours) was the area under the curve (AUC) generated from the dose - response curve. Tramadol was administered alone orally at doses of 3.16, 5.62, 10.0, 17.78, 31.62, 56.23 and 100.0 mg/kg, suspended in carboxymethyl cellulose. Gabapentin was administered alone at doses of 31.6, 56.2, 100.0, 177.8, 316.2 suspended in carboxymethyl cellulose (0.5%).

The area under the curve, for each tested mixture of components and for each of the components, was estimated.' Based on the additive effect of individual components, the area under the curve (AUC) was equivalent to the obtained sum. If the area under the curve from the combination was greater than the sum of the area under the curve (AUC) of individually administered medicaments, the result was considered as enhancement, and if it was equal, it was considered an additive analgesic effect. All of the values in the test meant at least six animals per experimental point. Observed values of the area under the curve (AUC) for each of the mixtures were compared to the expected value using the Student's t-test. The p value < 0.05 was considered statistically significant.

**Design of the study:** The antinociceptive effects caused by tramadol and gabapentin administered individually or in combination were studied.

First, each tramadol dose (3.16, 5.62, 10.0, 17.78, 31.62, 56.23, 100.0 mg/kg) was administered to six animals in order to obtain the corresponding Dose-Response Curve (DRC) and the doses of gabapentin alone were 31.6, 56.2, 100.0, 177.8, 316.2.

Doses used for the combination were 3.16, 5.62, 10.0 or 17.78, 31.62, 56.23, 100.0 mg/kg of tramadol and those used for gabapentin were 31.6, 56.2, 100.0, 177.8, 316.2 mg/kg. Thus, the possible synergistic interaction (a total of 24 combinations) was analyzed.

The synergism between gabapentin and tramadol was calculated with the synergistic interaction surface analysis and an isobolographic method (Tallarida et al., 1989). The area under the curve (AUC) was calculated for each of the combinations and for each of the components.

### Results:

Uric acid induced a complete dysfunction of the paw within 2.5 hours, corresponding to zero Functionality Rate (FR). Rats that were administered vehicle (0.5% methyl cellulose) did not present significant recovery of Functionality Rate during the 4-hours observation period.

**Antinociceptive effects of individually tested drugs.** Both drugs, gabapentin and tramadol, increased the area under de curve (AUC) in a dose-dependent manner.

Gabapentin (316.2 mg/kg) produced a total analgesic effect of 121.5 ± 30.3 area units (AUC); tramadol (56.23 mg/kg) produced a total analgesic effect of 207.7 ± 24.7 area units (AUC).

**Antinociceptive effects of combined tested drugs.** Fourteen of the gabapentin + tramadol combinations produced antinociceptive additive effects and 10 produced enhancement with 95% confidence limits (P<0.05).

The combination which produced the highest antinociceptive effects was gabapentin 316.2 mg/kg + tramadol 56.23 mg/kg, 329.2 ± 55.0 area units (AUC).

From these data, it is concluded that gabapentin is a potent enhancer of tramadol antinociception; the coadministration of gabapentin and tramadol produced a higher antinociceptive effect than that observed after individual doses; the enhanced antinociceptive effects were not accompanied by higher side effects; the fact that the gabapentin + tramadol combination produced higher enhancement effects was an interesting data, the consumption of gabapentin and tramadol was lower when both drugs are,administered together.

Example 2 shows the synergic activity of the combination in neuropathic pain.

### Example 2:

A clinical study was performed which object was to evaluate the efficacy and tolerance in individuals with neuropathic pain. It was a prospective, double-blind, randomized phase III study consisting of 3 phases/steps: Assessment, Treatment, Post-treatment. 50 individuals were included, and randomness was stratified in rate 1:1 in two treatment groups: Group 1: Individuals who had a diagnosis of diabetic neuropathy (25 individuals) and Group 2: Individuals who had a diagnosis of post-herpetic neuralgia. Similar treatment regimes were used.

Study population: Male and/or female individuals older than 18 years old, with diagnosis of diabetic neuropathy and/or post-herpetic neuralgia, who required the use of an analgesic, such as Tramadol, and an anticonvulsant, such as Gabapentin, for moderate to severe pain. A capsule was administered every 12 hours during a 30-days treatment.

### Results:

38 female individuals and 12 male individuals were hospitalized, average age was 55.5 years old, with previous signature of informed consent and meeting the inclusion criteria, the administration of the drug in study was started.

At Group 1: all patients had antecedents of type 2 DM with at least 15-years progression, with diagnosis of Distal peripheral polyneuropathy in 25 (100%) of included individuals, with symmetric and distal appearance on lower limbs, characterized by intense burning pain of nightly prevalence in 100% (25 individuals) of patients, hyperesthesia in 98% (24 individuals) of cases, clinical manifestations of at least 2-months progression. Table 1.

**Table 1. Diagnostic characteristics and clinical manifestations Group 1.**

| **Clinical manifestations and diagnosis** | **No. of patients** |
|---|---|
| Distal peripheral polyneuropathy | **25** |
| Intense pain | **25** |
| Hyperesthesia | **24** |

In the assessment of visual analog scale, 96% mentioned moderate to intense pain, the remaining 4% manifested an intense and nightly prevalence pain.

In Lans pain scale, an average score of 15 was reported.

During sensorial examination in 98% (24 individuals), it was determined allodynia in lower limbs and 24% of them presented an altered puncture threshold (low PT), establishing a total average score of 18 points, documenting that exist neuropathic mechanisms which account for pain.

At Group 2: 25 individuals with diagnosis of post-herpetic neuropathy were included, 21 female and 4 male. Table 4, average age of 66.2 years, who met inclusion criteria and signed the informed consent letter, in none of the cases co-existed active herpetic pathology, with antecedents of at least 1 month pain, after disappearance of vesicular lesions distinctive of Herpes Zoster, establishing the diagnosis of pain associated to zoster in 100% of cases, in 96% of cases (24 individuals) thoracic post-herpetic neuropathy was documented, and in the remaining 4% (1 individual) the appearance was Post-herpetic neuropathy at the trigeminal branch.

The examination with visual analog scale documented moderate pain in 46% of cases and severe pain en the remaining 4%.

In the assessment through Lans pain scale, an average score of 20 was reported, and in the sensorial examination 9.8% of the patients presented allodynia, the puncture threshold was a high PT.

### EFFICACY EVALUATION VISIT 3

The researcher determined the clinical response on the third visit by comparing the signs and symptoms with which the patients were registered on their admission; the clinical response was assessed as follows:
**Healed:** Resolution of signs and symptoms associated to active affection.
**Got better:** Incomplete resolution of signs and symptoms associated to affection but which do not require further therapy.
**Failed:** There is no response to therapy (it can also be designated if the individual discontinued the drug in study due to failure of treatment after a 48-hours therapy).
**Unable to evaluate:** Due to insufficient administration of the drug in study (48 hours or less), the individual did not get back to evaluations, etc.

During the course of the clinical study, the patients manifested important changes, both in emotional items and quality of life, in 88% of cases (44 individuals) gradual decrease in pain intensity with significant changes in the final visit was documented, even some patients requested to extend the time of study since they.expressed fear to present again the painful manifestations reported on their admission.

In the visual analog scale of moderate pain reported in both groups, 43 individuals presented a significant decrease until it was established in a mild pain, and at the end of the treatment the researcher determined healing, because resolution of signs and symptoms associated to active affection was documented, completely indicating reincorporation to their daily activities. In Lans evaluation, the score also had a significant decrease by obtaining a score less than 8. Table 2 and 3.

**TABLE 2. VISUAL ANALOG SCALE BOTH GROUPS**

| | Mild | Moderate |
|---|---|---|
| Group 1 | 23 | 2 |
| Group | 21 | 4 |

Inability to evaluate was documented in 3 individuals, because these patients did not present themselves to the final visit of the treatment in group 2; in group 2 all of the patients concluded the research study.

**TABLE 3. EFFICACY EVALUATION**

| | Group 1 | Group 2 |
|---|---|---|
| Healing | 19 | 21 |
| Got better | 3 | 4 |
| Failed | 0 | 0 |
| Unable | 3 | 0 |

Three (6% of cases) non-serious adverse events were reported, which deserved to change the schedule of administration, preferably post-prandial, those which were characterized by dizziness (1 event) and nausea (2 events) did not deserved neither to suspend the study therapy nor to receive concomitant therapy.

### CONCLUSIONS

Neuropathic pain is a very complex painful syndrome, with various symptoms and signs, which fluctuate over time in number and intensity. The pain results from the injury or disorder of the nervous system, whether at a peripheral or central branch or both, causing an disorder in the nervous transmission of pain, so it is unchained in the absence of recognizable stimulus. It is quite common in the clinical practice, being observed in a 2-40% of population. It is quite varied regarding' physiopathogenic mechanisms, topographies and clinical manifestations. It is common that patients with the same symptom refer distinct symptoms. Examples of syndromes with neuropathic pain are: cases owing to infection (post-herpetic neuralgia, neuralgia-associated HIV); metabolic' disorders (diabetic neuropathy); complex regional pain syndrome; trauma or entrapping of peripheral nerve (such as in trigeminal neuralgia or pain of the phantomlimb); cerebrovascular ischemic post-accident pain syndrome, nerve trunk trauma; some types related to neoplasia or to its treatment; disorders of the spinal cord such as multiple sclerosis.

Diabetes' mellitus is the most common endocrine disease; its incidence varies between 1 and 2%, of population. The most common form is the non-insulin-dependent diabetes mellitus (NIDDM). Neuropathic pain is one of the most common and troublesome complications of diabetic patients, affecting about 45% of the diabetics of more than 25 years of progression and more of 50% of diabetics patients older that 60 years. The pain may be presented en several of the distinct types of diabetic neuropathy; but it has a varying incidence and intensity extent and its nature is subjective. Although it is rarely a direct cause of mortality, it is indeed a cause of morbidity and it can affect almost any part of the nervous system.

### Peripheral neuropathy-

*Peripheral polyneuropathy.* It is the'most common form of appearance. Its location is often symmetrical and distal in MMII with a "sock" distribution and produces numbing, paresthesia, intense hyperesthesia and pain. The pain may be intense and burning such as in tabes dorsalis (psudotabes) and it often get worse at nights, being accompanied by hyperesthesia which make the patient unable to tolerate the rubbing of the sheets.

The affectation of the proprioceptive fibers causes disorders of the gait and artropathy (Charcot joint). In the physical examination' highlights the abolition of tendinous reflexes and loss of vibratory sensitivity.

*Proximal symmetrical polyneuropathy.* It is manly characterized by a strength loss affecting specially the pelvic girdle, among others.

The diabetic's neuropathic pain is often associated with mood repercussion, sleep disorders, emotional suffering, functional decay and disorders in social relations. Therefore, the life quality of patient considerably decrease. Nonetheless, only a minority of the patients who present diabetic neuropathy refers clinical symptomatology, and thus the absence of symptoms does not' discards the presence of neuropathy.

Post-herpetic neuralgia (PHN) is not a continuation of the herpes zoster, but a complication thereof.

The HZ is a disease caused by an DNA virus, characterized by unilateral radicular pain, which is often accompanied by vesicular rash, normally limited to the dermatome innervated by the same sensitive ganglion, auto-limited in time to about three weeks, being often resolved without complications. By PHN it is understood the continuous pain along a nerve and its ramifications, commonly without inflammatory phenomena, which occurs during more than one month once the skin lesions disappear; other authors consider it from two months.

Multiple therapies in treatment of neuropathic pain have successfully been used. From the comparative and randomized clinical tests, stand out those performed in diabetic neuropathies and post-herpetic neuralgias. But its efficacy and security in other neuropathic painful syndromes of different etiologies has also been checked.

Pure agonist opioids. In Germany since 1977, it has been shown to be valid in treatment of severe and moderate pain of all origins. In the last few years, their use for analgesia has been increasing in other countries. In. 1966, the World Health Organization recommended their use in a second step for treatment of tumor pain.

It is concluded that the neuropathic pain, intense and incapacitating, is often resistant to customary analgesic treatments, for which reason the association with an anticonvulsant drug such as Gabapentin, which acts over specific receptors involved in the genesis and maintenance of hyper-excitability, and Tramadol, a pure agonist opioid analgesic for the management of severe and moderate pain of all origins, has shown to be secure and efficacious in treatment of neuropathic pain.

The invention has been described sufficiently such that a person with middle knowledge in the art is able to reproduce and obtain the results mentioned in the present invention. However, any person skilled in the art concerned with the present invention can be able to make modifications not described in the present application; nonetheless, if for the application of such modifications in a certain composition or in the manufacture process thereof, the subject-matter claimed in the following claims is required, said structures shall be comprised within the scope of the invention.

Having thus described the present invention, it is considered as a novelty, and therefore, it is related as property that contained in the following claims:

## Claims

1. Pharmaceutical composition for treatment of sharp and chronic pain, **characterized in that** it comprises a combination of an analgesic and an anticonvulsant.

2. Pharmaceutical composition as claimed in claim 1, **characterized in that** the analgesic medicament is Tramadol and/or any of its salts, and the anticonvulsant medicament is Gabapentin and/or any of its salts.

3. A pharmaceutical composition in accordance with claim 2, **characterized in that** Tramadol is present in a rate with respect to the total weight of the formulation of from 3 to 8.3% and Gabapentin is present in a rate with respect to the total weight of the formulation of from 16 to 83%.

4. A pharmaceutical composition in accordance with claim 2, **characterized in that** Tramadol is present preferably in a range of from 4 to 5% and Gabapentin is present in a rate with respect to the total weight of the formulation in a range of from 40 to 60%.

5. A pharmaceutical composition in accordance with claim 2, **characterized in that** Tramadol is present preferably in a rate of 4.16% and Gabapentin is present in a rate with respect to the total weight of the formulation of 50%.

6. A pharmaceutical composition in accordance with claim 2, wherein Tramadol is dosed in a range of from 20 to 50 mg per dosage unit and Gabapentin is dosed in a range of from 100 to 500 mg per dosage unit.

7. A pharmaceutical composition in accordance with claim 2, wherein Tramadol is dosed in a range of from 20 to 30 mg per dosage unit and Gabapentin is dosed in a dosage range of from 200 to 400 mg.

8. A pharmaceutical composition in accordance with claim 2, wherein Tramadol is dosed in a dose of 25 mg per dosage unit and Gabapentin is dosed in a dose of 300 mg per dosage unit.
